(19) Europäisches Patentamt
European Patent Office
Office européen des brevets

(11) EP 4 462 118 A1

(12) EUROPEAN PATENT APPLICATION

(43) Date of publication:
13.11.2024 Bulletin 2024/46

(21) Application number: 24171152.2

(22) Date of filing: 18.04.2024

(51) International Patent Classification (IPC):
G01N 33/48 (2006.01)   G01N 33/72 (2006.01)
G01N 30/50 (2006.01)   G01N 30/88 (2006.01)

(52) Cooperative Patent Classification (CPC):
G01N 30/50; G01N 33/48; G01N 33/721;
G01N 2030/8822

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 21.04.2023 JP 2023070465

(71) Applicant: ARKRAY, Inc.
Kyoto-shi, Kyoto 601-8045 (JP)

(72) Inventor: ISHIKAWA, Kazuki
Kyoto, 602-0008 (JP)

(74) Representative: Dehns
10 Old Bailey
London EC4M 7NG (GB)

(54) **METHOD OF TREATING MEASUREMENT FLOW CHANNEL AND LIQUID CHROMATOGRAPHY DEVICE**

(57) A method of treating a measurement flow channel (40, 50), the method comprising performing a conditioning treatment by causing a conditioning liquid, in which albumin, casein, or skim milk is dissolved, to flow through a measurement flow channel (40, 50) of a liquid chromatography device (10) for analyzing hemoglobin in a specimen before analyzing the hemoglobin.

FIG.1

**Description**

BACKGROUND

Technical Field

The present invention relates to a method of treating a measurement flow channel used in hemoglobin analysis by liquid chromatography, and a liquid chromatography device.

Related Art

Hemoglobin in blood is a diagnostic material for various diseases. For example, HbA1c, which is glycosylated HbA, is a diagnostic index for diabetes, and HbA2 is a diagnostic index for thalassemia. These types of hemoglobin are measured in accordance with various measurement principles. For example, in cation exchange chromatography, peaks of various types of hemoglobin are separated, an area is calculated to calculate the ratio of the hemoglobin species, and this ratio is used as a measured value. This measured value is obtained based on absorbance obtained by irradiating an optical cell provided downstream of a liquid chromatography column with light having an absorption wavelength of hemoglobin. In order to diagnose diseases as described above with high accuracy, measurement of hemoglobin also requires high accuracy, and it is required to favorably separate peaks of hemoglobin.

In a liquid chromatography device, since a high pressure is applied to a column or an optical cell, a member having a flow channel for securing mechanical strength is often formed of a metal such as stainless steel. Since a protein component of a specimen may be nonspecifically adsorbed to a metal surface of the flow channel, measurement results may be affected. In view of this point, Japanese Patent Application Laid-Open (JP-A) No. 2017-227461 discloses that a column for liquid chromatography using a porous metal filter is subjected to conditioning with polylysine.

SUMMARY

Since a measurement flow channel becomes dirty while the hemoglobin analysis is repeated by high-performance liquid chromatography, it is necessary to periodically perform cleaning. However, in the hemoglobin analysis performed immediately after cleaning, there have been cases in which the degree of separation between adjacent peaks has, deteriorated. This is thought to be due to adsorption of hemoglobin to the clean metal surface of the measurement flow channel.

An object of a method of treating a measurement flow channel of the present disclosure is to suppress adsorption of hemoglobin to the measurement flow channel used in liquid chromatography and to improve the separability of adjacent peaks.

In the method of treating a measurement flow channel of the disclosure, a conditioning treatment by causing a conditioning liquid, in which albumin, casein, or skim milk is dissolved, to flow through a measurement flow channel of a liquid chromatography device for analyzing hemoglobin in a specimen, is performed before analyzing the hemoglobin.

According to the treatment method of the disclosure, the separability of adj acent peaks of hemoglobin can be improved.

BRIEF DESCRIPTION OF THE DRAWINGS

Exemplary embodiments will be described in detail based on the following figures, wherein:

Fig. 1 is a schematic diagram illustrating an outline of a liquid chromatography device of a first embodiment;
Fig. 2 is a schematic diagram illustrating an outline of a liquid chromatography device of a second embodiment;
Fig. 3 shows an example of a hemoglobin analysis chromatogram;
Fig. 4 is an imaginary hemoglobin analysis chromatogram for describing a degree of separation between two adjacent peaks;
Fig. 5 is a hemoglobin analysis chromatogram showing a comparison before and after cleaning an optical cell;
Fig. 6 is a graph showing the transition of an HbA2 peak value after optical cell cleaning;
Fig. 7 is a chromatogram obtained by comparing the measurement immediately after optical cell cleaning and the 300th measurement thereafter;
Fig. 8 is a graph showing the transition of the degree of separation between an HbA0 peak and an HbA2 peak after optical cell cleaning;
Fig. 9 is a graph obtained by comparing the effects of a conditioning treatment with various conditioning liquids in terms of the HbA2 peak value; and

Fig. 10 is a graph obtained by comparing the effects of a conditioning treatment with various conditioning liquids in terms of the degree of separation between the HbA0 peak and the HbA2 peak.

DETAILED DESCRIPTION

Hemoglobin has a complicated three-dimensional structure, and thus has a strong charge-sensitive and hydrophobic adsorption action, and easily binds to the metal surface used for piping of a measurement flow channel, a detector, and the like. At the time of hemoglobin analysis, adjacent peaks may be fused with each other, and a favorable separability cannot be obtained in some cases. This is because hemoglobin in the specimen comes into contact with and is adsorbed by a member containing metal in the measurement flow channel in the process in which the specimen is introduced into an analyzer and passes through the detector via the column, and the width of the detection peak is enlarged.

Since the column requires pressure resistance, it is difficult to replace the material of the column with resin, and metal including iron such as stainless steel is inevitably used. Replacement of piping with a resin such as polyether ether ketone (PEEK) has progressed, but the resin cannot be used in the detector, and there is a portion where iron atoms are exposed on an inner surface thereof.

However, in a method of treating a measurement flow channel of the present embodiment, by performing a conditioning treatment of causing a conditioning liquid, in which albumin, casein, or skim milk is dissolved, to flow through a measurement flow channel of a liquid chromatography device for analyzing hemoglobin in a specimen before analyzing the hemoglobin, non-specific binding between iron and hemoglobin in the flow channel can be suppressed, thereby improving the separability of adjacent peaks.

The measurement flow channel subjected to the conditioning treatment with the conditioning liquid is a flow channel through which the specimen flows. Specifically, the measurement flow channel is a flow channel through which the specimen passes until the specimen passes through an optical cell constituting a part of a hemoglobin detection unit via a column. For example, a flow channel connecting an injector for introducing a specimen into a column and the column provided downstream of the injector, a flow channel connecting the column and a detection unit provided downstream of the column, and the detection unit may be surface-treated with a surface treatment liquid.

The term "conditioning" mentioned in the present disclosure means that a liquid (i.e., the conditioning liquid described in the present disclosure) containing a protein irrelevant to an object to be measured (specifically, hemoglobin) of a specimen is brought into contact in advance with a portion where the specimen contacts in the measurement flow channel, so that the irrelevant protein is non-specifically adsorbed to the surface. By this conditioning, non-specific adsorption of hemoglobin, which is the object to be measured in the specimen, to the surface of the measurement flow channel is prevented by the irrelevant protein non-specifically adsorbing in advance to the measurement flow channel.

In the conditioning liquid, albumin, casein, or skim milk is dissolved as the protein irrelevant to hemoglobin or a substance containing the protein. As long as at least one of albumin, casein, or skim milk is dissolved, a substance other than these may also be dissolved. As the albumin, bovine serum albumin (BSA) or human serum albumin (HSA) can be used, but it is preferable to use BSA as a more irrelevant protein in the sense of measuring human hemoglobin.

The concentration of albumin, casein, or skim milk in the conditioning liquid is from 0.5 to 5% by mass, preferably from 1 to 3% by mass, and more preferably from 1.5 to 2.5% by mass. Note that, in the case in which two or more of albumin, casein, or skim milk are dissolved in the conditioning liquid, the concentration mentioned above means the sum of the concentrations of these two components or more. As the solvent used for the conditioning liquid, purified water is preferable for albumin and skim milk, and phosphate buffered saline (PBS) is preferable for casein that is sparingly soluble in water.

The conditioning treatment of the measurement flow channel may be performed by bringing the conditioning liquid into contact with the inner surface of the measurement flow channel. For example, the inside of the measurement flow channel may be immersed in the conditioning liquid, or the conditioning liquid may pass through the measurement flow channel. Note that it is preferable to treat the entire inner surface of the measurement flow channel with the conditioning liquid, but in the case in which the portion of the measurement flow channel where the metal is exposed is known, only the portion may be subjected to the conditioning treatment. For example, in the case in which the metal is exposed only on the inner surface of the column in the measurement flow channel, only the inner surface of the column may be subjected to the conditioning treatment. Moreover, it is preferable to subject the entire portion where the metal is exposed in the inner surface of the measurement flow channel to the conditioning treatment, but it is only necessary to subject only a part of the portion where the metal is exposed to the conditioning treatment.

Note that the measurement flow channel of the liquid chromatography device is cleaned at an appropriate timing, and it is effective to use a cleaning liquid containing hypochlorous acid for this cleaning. However, immediately after the measurement flow channel is cleaned with the cleaning liquid containing hypochlorous acid, the metal portion of the measurement flow channel is exposed, and hemoglobin is likely to cause non-specific adsorption at the time of the subsequent hemoglobin measurement. Therefore, it is preferable to perform the conditioning treatment of the disclosure after the measurement flow channel is cleaned with hypochlorous acid.

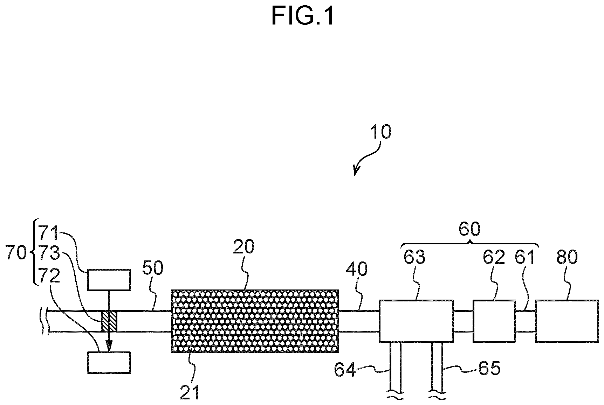

The method of treating a measurement flow channel of the disclosure is performed, for example, on a liquid chromatography device 10 schematically illustrated as in the first embodiment illustrated in Fig. 1. In the liquid chromatography device 10, measurement flow channels 40, 50 are connected to an upstream side and a downstream side, respectively, of a column 20 into which a filler 21 as a carrier suitable for hemoglobin to be analyzed is filled. An injector 60 for introducing a liquid into the measurement flow channel 40 is provided further upstream of the measurement flow channel 40 on the upstream side. An eluent supply path 61 to which an eluent is supplied is located the furthest upstream in the injector 60, and an eluent storage tank 80 storing an eluent is located upstream thereof. Note that the eluent storage tank 80 is not limited to a tank shape, and may be a disposable pack in which an eluent is sealed.

On the other hand, a pump 62 delivering an eluent downstream is located downstream of the eluent supply path 61. An introduction unit 63, which joins a conditioning liquid flow channel 64 causing the conditioning liquid to flow into the measurement flow channel 40 on the upstream side and a specimen flow channel 65 causing a specimen to flow into the measurement flow channel, is located downstream of the pump 62.

The eluent storage tank 80 is divided into a plurality of regions (or a plurality of packs), and a plurality of types of eluents are stored so as not to be mixed with each other. The pump 62 can mix a plurality of types of eluents stored in the eluent storage tank 80 at an arbitrary ratio and deliver the mixture downstream. The measurement flow channel 50 downstream of the column 20 is provided with a light source 71 which emits light (indicated by an arrow in the drawing) including the absorption wavelength of hemoglobin into the measurement flow channel 50, an optical cell 73 which is provided with a flow channel connected to the measurement flow channel 50 and through which light emitted from the light source 71 passes, and a detector 72 which receives the light passing through the optical cell 73. In the measurement flow channel 50, the portion where hemoglobin is detected by the light source 71, the optical cell 73, and the detector 72 is the detection unit 70.

In one embodiment the invention provides a liquid chromatography device (10) having a measurement flow channel (40, 50) and analyzing hemoglobin in a specimen by having the specimen flow through the measurement flow channel (40, 50),

wherein the measurement flow channel (40, 50) is subjected to a conditioning treatment in advance before measurement of the hemoglobin, with a conditioning liquid in which albumin, casein, or skim milk is dissolved.

The device is suitable for analyzing hemoglobin in a specimen. Before such analysis the measurement flow channel has been subjected to a conditioning treatment.

In preferred embodiments the liquid chromatography device (10) comprises a supply device (90) supplying a conditioning liquid, in which albumin, casein, or skim milk is dissolved, to the measurement flow channel (40, 50). The supply device is for supplying the conditioning medium. Such a device may be used in methods of the invention.

In the liquid chromatography device 10 of the first embodiment, an appropriate container (for example, a syringe or the like) containing the conditioning liquid is connected to the conditioning liquid flow channel 64 and the conditioning liquid is injected into the flow channel every time the conditioning treatment is performed. As a result, in the liquid chromatography device 10 having the measurement flow channels 40, 50 and analyzing hemoglobin in a specimen by having the specimen flow through the measurement flow channel, the measurement flow channels 40, 50 are subjected to a conditioning treatment in advance before measurement of the hemoglobin, with a conditioning liquid in which albumin, casein, or skim milk is dissolved.

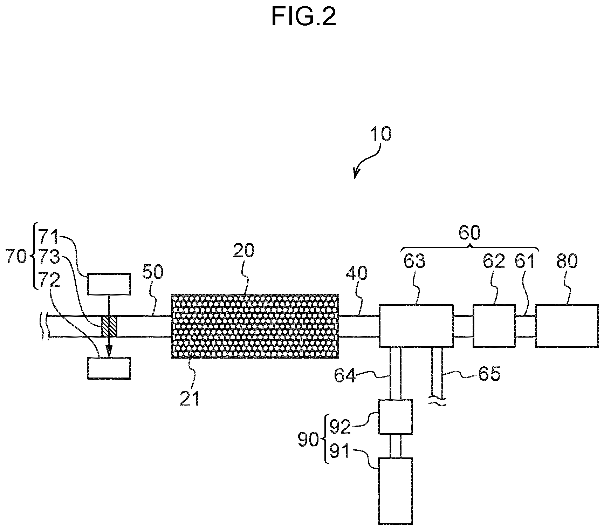

On the other hand, the liquid chromatography device 10 of a second embodiment illustrated in Fig. 2 is the same as the liquid chromatography device 10 of the first embodiment for the column 20 into which the filler 21 is filled, the measurement flow channels 40, 50 located upstream and downstream of the column 20, respectively, the eluent supply path 61, the pump 62, the introduction unit 63, the conditioning liquid flow channel 64, the injector 60 having the specimen flow channel 65, the light source 71, and the detection unit 70 having the optical cell 73 and the detector 72. However, the liquid chromatography device 10 of the second embodiment is different from the liquid chromatography device 10 of the first embodiment in that a supply device 90 supplying a conditioning liquid in which albumin, casein, or skim milk is dissolved to the measurement flow channel is provided upstream of the conditioning liquid flow channel 64.

The supply device 90 includes a conditioning liquid storage tank 91 storing the conditioning liquid and a pump 92 delivering the conditioning liquid to the conditioning liquid flow channel 64. The pump 92 of the supply device 90 is controlled by a control device (not illustrated) similarly to the pump 62 of the injector 60. As a result, the conditioning treatment of the measurement flow channels 40, 50 can be executed at a predetermined timing, for example, after a predetermined number of measurements or at predetermined time intervals.

In this example, the specimen introduced into the introduction unit 63 of the injector 60 through the specimen flow channel 65 flows through the measurement flow channel 40 on the upstream side, the column 20, the measurement flow channel 50 on the downstream side, and the optical cell 73 in this order. The metal is also exposed on the inner surface of the flow channel provided in the optical cell 73. Therefore, by subjecting the flow channel of the optical cell 73 through which the specimen flows to the conditioning treatment in advance, non-specific adsorption to the measurement flow channel 50 is prevented in the process in which hemoglobin contained in the specimen reaches the detection unit 70.

After the conditioning treatment, the eluent is delivered to the measurement flow channel 40, and then the specimen is introduced from the specimen flow channel 65 into the introduction unit 63 in the injector 60. Then, an eluent having an appropriate composition suitable for each hemoglobin fraction is sequentially delivered to the measurement flow channel 40. The specimen introduced into the measurement flow channel 40 together with the eluent is delivered to the column 20, and hemoglobin contained in the specimen is separated into hemoglobin fractions. The absorbance of the separated hemoglobin fraction is measured by the detection unit 70 provided in the measurement flow channel 50 downstream of the column 20. Then, a chromatogram showing the absorbance with respect to the elapsed time from the time when the specimen was introduced into the measurement flow channel 40 is obtained.

The invention also provides a method of analyzing hemoglobin using the conditioning treatment of the invention. Thus, in one embodiment the present invention provides a method of analyzing hemoglobin in a specimen, comprising

a) treating a measurement flow channel (40, 50) in a liquid chromatography device (10) by performing a conditioning treatment by causing a conditioning liquid, in which albumin, casein, or skim milk is dissolved, to flow through the measurement flow channel of said liquid chromatography device, and
b) analyzing the hemoglobin in said specimen in said liquid chromatography device. The hemoglobin and conditioning treatment is as described herein.

In a preferred aspect the conditioning treatment is performed after the measurement flow channel (40, 50) is cleaned with hypochlorous acid. Therefore the above method may include an additional step, i.e. step a) cleaning a measurement flow channel (40, 50) with hypochlorous acid. This is then followed by steps a) and b) above.

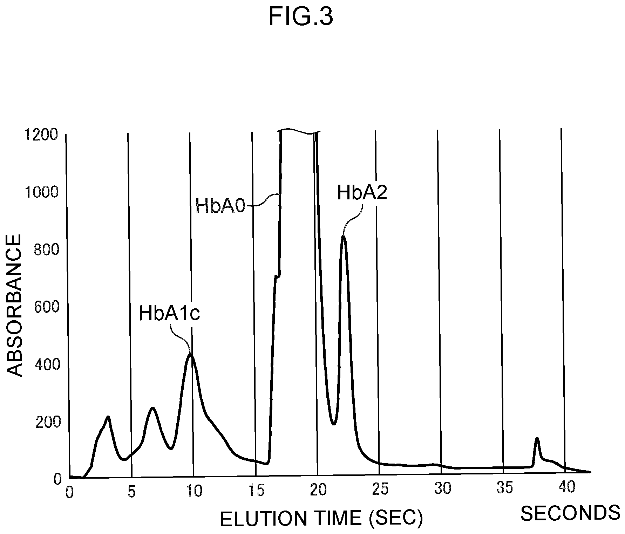

An example of a chromatogram in the case in which the hemoglobin is analyzed is shown in Fig. 3. In this chromatogram, the HbA1c peak appears around an elution time of 9.8 seconds, the HbA0 peak appears around an elution time of 18.8 seconds, and the HbA2 peak appears around an elution time of 22.2 seconds.

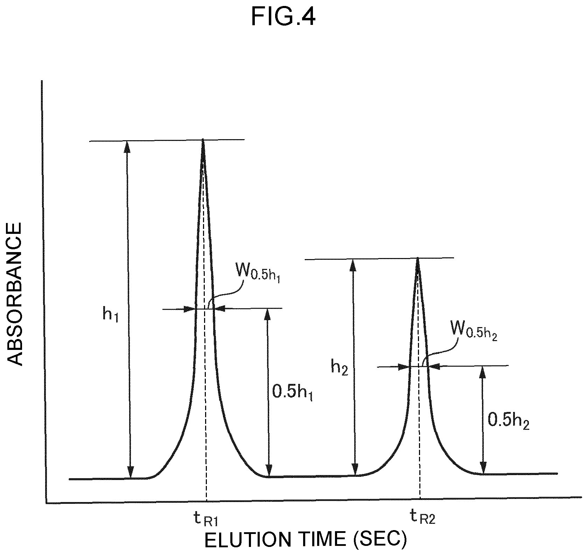

As in an imaginary hemoglobin analysis chromatogram shown in Fig. 4, two peaks to be analyzed are assumed. The vertical axis and the horizontal axis in the chromatogram in Fig. 4 represent the absorbance and the elution time (sec) as in Fig. 3, respectively. The elution time corresponding to the vertex of the peak (hereinafter, referred to as "first peak") on the left side in the drawing is designated as $t_{R1}$, and the elution time corresponding to the vertex of the peak (hereinafter, referred to as "second peak") on the right side in the drawing is designated as $t_{R2}$. Moreover, providing that the height of the first peak is designated as hi, and the height of the second peak is designated as $h_2$, the width (hereinafter, referred to as "half-value width" (unit: time (sec)) of the peak at the height 0.5hi in the first peak is designated as $W_{0.5h1}$, and the half-value width at the height $0.5h_2$ in the second peak is designated as $W_{0.5h2}$. At this time, the degree of separation R between the first peak and the second peak is defined by the following formula.

$$[\text{Math. 1}]$$

$$R = \frac{t_{R2} - t_{R1}}{W_{0.5h1} + W_{0.5h2}}$$

That is, the larger the peak-to-peak distance (in other words, the time difference in which a peak occurs) defined by "$t_{R2} - t_{R1}$" in the above mathematical formula is, and the smaller the width of each peak is, the higher the degree of separation between two peaks.

Examples

(1) Influence of Measurement Flow Channel Cleaning

In the hemoglobin analysis by the liquid chromatography device, the influence of washing the measurement flow channel on the degree of separation of the peak was examined. Specifically, first, in the detection unit, a pipe connection portion from the column was removed from an introduction port of the optical cell made of stainless steel. Next, 2 mL of a 0.5% by mass sodium hypochlorite solution was injected from the introduction port of the optical cell using a syringe, the measurement flow channel in the optical cell was filled with the sodium hypochlorite solution, and the optical cell was allowed to stand still in this state for 5 minutes. As a result, it is presumed that the protein adsorbed to the measurement flow channel in the optical cell is oxidatively decomposed. Thereafter, the pipe connection portion from the column was attached to the introduction port of the optical cell 73, a sufficient amount of the eluent was delivered, the sodium hypochlorite solution in the optical cell was discharged, and the eluent was replaced.

The hemoglobin analysis of human blood as a specimen was performed before and after cleaning the measurement flow channel. The hemoglobin analysis was performed as follows.

(1-1) Eluent

The following eluents A to C were prepared as eluents. As eluent A, an aqueous solution of 1.44% by mass of sodium dihydrogen phosphate dihydrate and 0.16% by mass of disodium hydrogen phosphate was adjusted to a pH of 5.08. As eluent B, an aqueous solution of 0.02% by mass of sodium dihydrogen phosphate dihydrate and 0.50% by mass of disodium hydrogen phosphate was adjusted to a pH of 8.0. As eluent C, an aqueous solution of 0.12% by mass of sodium dihydrogen phosphate dihydrate and 0.33% by mass of disodium hydrogen phosphate was adjusted to a pH of 6.82.

(1-2) Analysis Method

First, eluent A was allowed to flow through the column to be equilibrated, and then a predetermined amount of the diluted specimen was introduced into the column. Then, eluent A was allowed to flow for 13 seconds to elute HbF and HbA1c. Next, HbA0 was eluted by allowing a liquid obtained by mixing eluent A and eluent C at a ratio of 1 : 9 to flow for 5 seconds. Then, eluent C was allowed to flow for 17 seconds to elute HbA2. Next, the eluent B was allowed to flow for 2 seconds to elute all hemoglobin remaining in the analytical column. Finally, eluent A was allowed to flow for 5 seconds. The measurement wavelength in the detection unit was set to 420 nm, and a chromatogram was prepared from the obtained absorbance.

(1-3) Measurement Results

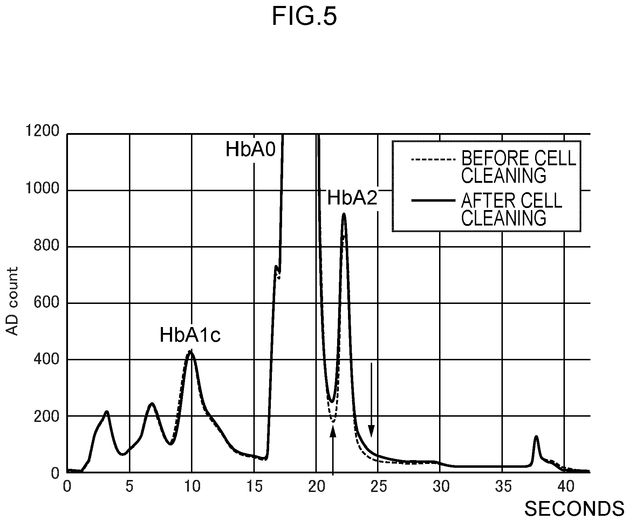

The measurement results were as shown in Fig. 5. That is, in the chromatogram after optical cell cleaning, so-called tailing in which the base of the peak is widened occurred in both the HbA0 peak and the HbA2 peak as indicated by arrows in the drawing.
Moreover, the ratio of the HbA2 peak to the entire peaks in the chromatogram (hereinafter referred to as "A2 ratio") was 2.66% before cleaning and 2.96% after cleaning. This is considered to be because the A2 ratio after cleaning was increased due to the influence of tailing. Accordingly, the degree of separation between the HbA0 peak and the HbA2 peak (hereinafter, simply referred to as "degree of separation") was 0.767 before cleaning, whereas the degree of separation was 0.713 after cleaning, and deterioration due to cleaning was confirmed.

(2) Influence of Measurement after Cleaning

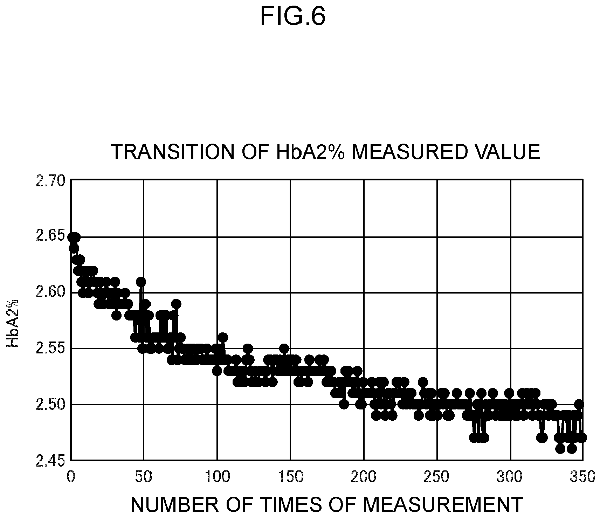

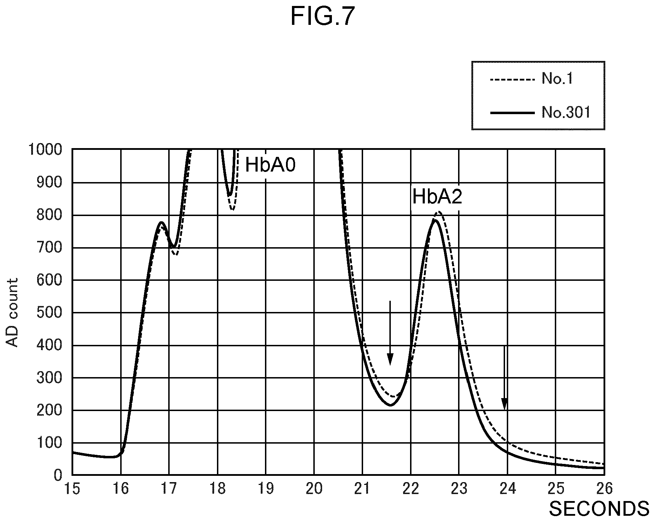

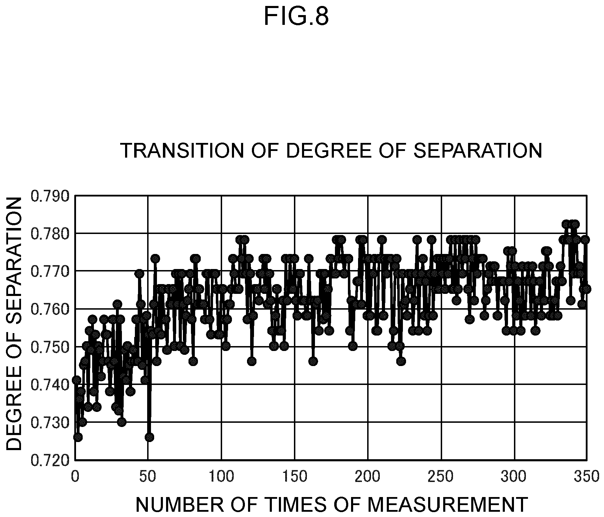

Next, after cleaning the measurement flow channel, the influence of repeating hemoglobin analysis on the degree of separation of the peak was examined. Specifically, the measurement flow channel in the optical cell was cleaned and then replaced with an eluent in the same manner as in the above (1), and then the same measurement as in (1) was repeated using a specimen different from the specimen used in (1) to observe the A2 ratio (Fig. 6) and the degree of separation (Fig. 8).
As a result, as shown in Fig. 6, the A2 ratio decreased as the measurement was repeated. This is presumed to be a result of suppressing non-specific adsorption of hemoglobin at the time of measurement as the measurement flow channel is coated with hemoglobin protein while the measurement is repeated. Moreover, Fig. 7 in which the hemoglobin analysis chromatograms of the first measurement after cleaning and the 301st measurement are displayed in an overlapping manner shows that tailing is suppressed by repeating the measurement. Thereby, as shown in Fig. 8, it was also shown that the degree of separation was improved by repeating the measurement.

(3) Discussion

From the results of the above (1), the principles of the occurrence of tailing, fluctuation of the A2 ratio, and deterioration of the degree of separation are considered as follows. In the liquid chromatography device of the disclosure, absorbance is determined in the case in which hemoglobin separated by the column passes through the optical cell, and a chromatogram is thereby drawn. The optical cell is made of a stainless steel material, and it is generally known that proteins are adsorbed to stainless steel. Therefore, it is considered that part of the hemoglobin molecules passing through the optical cell advance in the flow channel while repeating adsorption and desorption on the stainless steel surface.
Then, since the adsorbed hemoglobin molecules are eluted slowly, it is considered that this appears in the chromatogram as tailing. The tailing of the HbA0 peak is considered to be remarkable because the peak is large and the number of molecules is large, so that adsorption is likely to occur. That is, it is considered that as a result of tailing occurring at the HbA0 peak, the degree of separation deteriorated, and the A2 ratio was calculated to be larger than the actual value as the degree of separation had deteriorated.

On the other hand, from the results of the above (2), it is considered that by repeating the hemoglobin measurement, the protein in the blood sample is adsorbed onto the stainless steel surface of the optical cell, and the number of sites where the hemoglobin molecules can be adsorbed gradually decreases, whereby the adsorption of hemoglobin is suppressed, and as a result, tailing is also suppressed. That is, it is considered that tailing occurs in a new optical cell or an optical cell immediately after cleaning, and tailing does not occur in an optical cell to which protein has already been adsorbed by repeating measurement.

In a new optical cell or an optical cell immediately after cleaning, as described above, tailing is likely to occur and the degree of separation is likely to deteriorate, so that it is difficult to accurately measure the A2 ratio. Therefore, as suggested from the results of the above (2), it is necessary to stabilize the measurement flow channel of the optical cell before measurement of the specimen. For this stabilization, measurement may be repeated using the specimen to be measured, but repeating 300 or more measurements is not realistic from the viewpoint of time and cost.

Whether a similar effect can be obtained by a conditioning treatment in which a protein solution irrelevant to a measurement system is allowed to flow in the measurement flow channel of the optical cell instead of stabilization of the measurement flow channel by repeating the measurement is verified below.

(4) Effect of Conditioning Treatment

(4-1) Conditioning Liquid

The materials shown in Table 1 below were dissolved in purified water to a final protein concentration of 2% by mass, thereby preparing a conditioning liquid. However, since casein is sparingly soluble in water, the concentration of casein was adjusted to the same concentration as other caseins with PBS (composition: sodium chloride 8 g/L, potassium chloride 0.2 g/L, disodium hydrogen phosphate 1.44 g/L, potassium dihydrogen phosphate 0.24 g/L, pH: 7.4). As "BSA" of "Solution 1", "BOVINE SERUM ALBUMIN" (Sigma-Aldrich, A7906-50G) was used. As "HSA" of "Solution 2", "Albumin, from human serum" (Sigma-Aldrich, A1653-10G) was used. As the "casein" of "Solution 3", "Casein, derived from milk" (FUJIFILM Wako Pure Chemical Corporation, 030-01505) was used. As the "skim milk" of "Solution 4", "Skim milk powder" (FUJIFILM Wako Pure Chemical Corporation, 190-12865) was used. An "accuracy control substance" used as a material of "Solution 5" was a hemoglobin solution having a known concentration used for measurement accuracy control of a liquid chromatography device, and specifically, "ADAMS A1c Control" (ARKRAY, Inc., 71285) was used. The blood cells and whole blood used as the materials of "Solution 7" and "Solution 8" were frozen at -80°C once and then hemolyzed.

[Table 1]

| Conditioning liquid | Material | Contained main protein |
|---|---|---|
| Solution 1 | BSA | Albumin (bovine) |
| Solution 2 | HSA | Albumin (human) |
| Solution 3 | Casein | Casein |
| Solution 4 | Skim milk | Lactoferrin, albumin, casein, lactophorin, keratin |
| Solution 5 | Accuracy control substance | Hemoglobin |
| Solution 6 | Plasma | Albumin, globulin, fibrinogen |
| Solution 7 | Blood cell | Hemoglobin |
| Solution 8 | Whole blood | Albumin, globulin, fibrinogen, hemoglobin |

(4-2) Evaluation Method

Each conditioning liquid in Table 1 was evaluated as follows. First, as in the above (1), the measurement flow channel in the optical cell was cleaned and then replaced with an eluent. Thereafter, the pipe connection portion from the column was removed again from the introduction port of the optical cell. Next, 2 mL of each conditioning liquid was injected from the introduction port of the optical cell using a syringe, the measurement flow channel in the optical cell was filled with the conditioning liquid, and the optical cell was allowed to stand still in this state for 5 minutes.

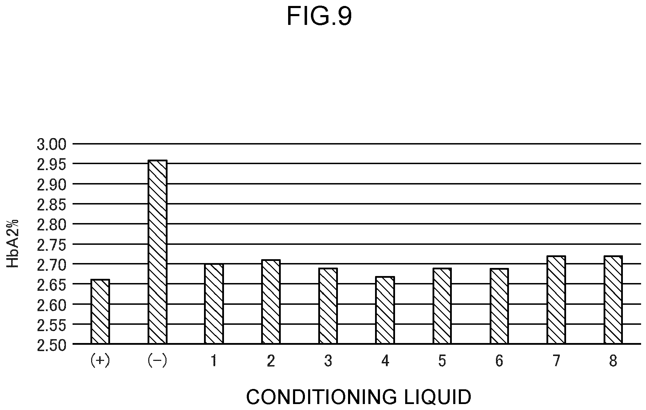

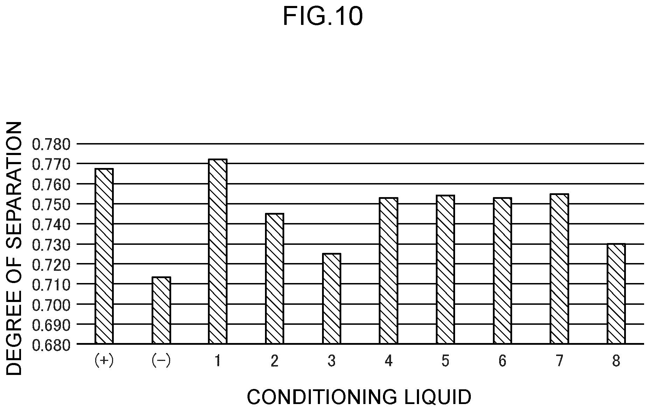

Thereafter, the pipe connection portion from the column was attached to the introduction port of the optical cell 73, a sufficient amount of the eluent was delivered, the conditioning liquid in the optical cell was discharged, and the eluent was replaced. In this state, the blood sample was measured in the same manner as in (1), and the results of measuring the A2 ratio and the degree of separation are shown in Figs. 9 and 10, respectively. Note that, in respective drawings, the results of measurement in the optical cell before cleaning are shown as a positive control (indicated as "(+)" in the drawing), and the results of measurement without the conditioning treatment after cleaning are shown as a negative control (indicated as "(-)" in the drawing).

From the results of the A2 ratio shown in Fig. 9, it was shown that tailing was sufficiently prevented in any conditioning liquid as in the case before cell cleaning. Also regarding the results of the degree of separation shown in Fig. 10, it was shown that the degree of separation was improved in any conditioning liquid as compared with the case in which the conditioning treatment was not performed. In particular, BSA showed a degree of separation higher than that before cell cleaning, and it was shown that the results of the conditioning treatment were particularly favorable.

Note that, for the accuracy control substance, blood cells, and whole blood, the effect of preventing tailing and improving the degree of separation was observed, but since hemoglobin is present as a protein, there is a concern that the measurement results may be affected in a measurement system in which the object to be measured is hemoglobin, and thus it is considered that the use thereof should be avoided. Furthermore, both HSA and plasma are substances derived from human blood, and it cannot be denied that HSA and plasma contain a trace amount of hemoglobin, and thus, it is better to avoid use of HSA and plasma for the same reason. From the above, it has been found that BSA is the most excellent material of the conditioning liquid, and skim milk and casein can also be used.

(5) Conditions for Use of BSA

Finally, the conditions for use of BSA, which was considered to be the most excellent as a material of the conditioning liquid in the above (4), were examined.

(5-1) Concentration

BSA was dissolved in purified water at various concentrations shown in Table 2 below to prepare a conditioning liquid. The prepared conditioning liquid was subjected to a conditioning treatment in the same manner as in the above (4-2), then the blood sample was measured in the same manner as in the above (1), and the A2 ratio and the degree of separation were measured. The results are also shown in Table 2 below. "(+)" and "(-)" in the table are the same as in the above (4-2).

[Table 2]

| BSA concentration (% by mass) | A2 ratio (%) | Degree of separation |
|---|---|---|
| (+) | 2.66 | 0.767 |
| (-) | 2.96 | 0.713 |
| 0.0001 | 2.92 | 0.720 |
| 0.0002 | 2.81 | 0.741 |
| 0.0003 | 2.69 | 0.751 |
| 0.0005 | 2.66 | 0.765 |
| 0.001 | 2.64 | 0.759 |
| 0.01 | 2.64 | 0.757 |
| 0.1 | 2.64 | 0.758 |
| 1 | 2.65 | 0.763 |
| 2 | 2.66 | 0.758 |

From the above results, it has been found that when the BSA concentration is higher, tailing is less likely to occur, and the degree of separation is improved.

(5-2) Treatment Time

BSA was dissolved in purified water at a concentration of 0.0002% to prepare a conditioning liquid. The prepared conditioning liquid was subjected to a conditioning treatment in the same manner as in the above (4-2) except that the treatment time was set as shown in the following Table 3, then the blood sample was measured in the same manner as in the above (1), and the A2 ratio and the degree of separation were measured. The results are also shown in Table 3 below. "(+)" and "(-)" in the table are the same as in the above (4-2).

[Table 3]

| Treatment time (min) | A2 ratio (%) | Degree of separation |
|---|---|---|
| (+) | 2.66 | 0.767 |
| (-) | 2.96 | 0.713 |
| 0.5 | 2.92 | 0.708 |
| 5 | 2.74 | 0.749 |
| 30 | 2.69 | 0.763 |
| 105 | 2.66 | 0.775 |

From the above results, it has been found that when the treatment time is longer, tailing is less likely to occur, and the degree of separation is improved.

Industrial Applicability

The invention can be used for hemoglobin analysis by a liquid chromatography device.

## Claims

1. A method of treating a measurement flow channel (40, 50), the method comprising performing a conditioning treatment by causing a conditioning liquid, in which albumin, casein, or skim milk is dissolved, to flow through the measurement flow channel (40, 50) of a liquid chromatography device (10) for analyzing hemoglobin in a specimen, before analyzing the hemoglobin.

2. The method according to claim 1, wherein the hemoglobin includes hemoglobin A0 and hemoglobin A2.

3. The method according to claim 1 or claim 2, wherein the albumin is bovine serum albumin.

4. The method according to any one of claims 1 to 3, wherein the conditioning treatment is performed after the measurement flow channel (40, 50) is cleaned with hypochlorous acid.

5. A method of analyzing hemoglobin in a specimen, comprising

    a) treating a measurement flow channel (40, 50) in a liquid chromatography device (10) by performing a conditioning treatment by causing a conditioning liquid, in which albumin, casein, or skim milk is dissolved, to flow through the measurement flow channel of said liquid chromatography device, and
    b) analyzing the hemoglobin in said specimen in said liquid chromatography device.

6. The method according to claim 5, wherein the hemoglobin includes hemoglobin A0 and hemoglobin A2.

7. The method according to claim 5 or claim 6, wherein the albumin is bovine serum albumin.

8. The method according to any one of claims 1 to 3, wherein the conditioning treatment is performed after the measurement flow channel (40, 50) is cleaned with hypochlorous acid.

9. A liquid chromatography device (10) having a measurement flow channel (40, 50) suitable for analyzing hemoglobin in a specimen by having the specimen flow through the measurement flow channel (40, 50),
wherein the measurement flow channel (40, 50) has been subjected to a conditioning treatment in advance before measurement of the hemoglobin, with a conditioning liquid in which albumin, casein, or skim milk is dissolved.

10. A liquid chromatography device (10) according to claim 9, wherein the liquid chromatography device (10) comprises a supply device (90) for supplying a conditioning liquid, in which albumin, casein, or skim milk is dissolved, to the measurement flow channel (40, 50).

FIG.1

FIG.2

# FIG.3

FIG.4

# FIG.5

# FIG.6

TRANSITION OF HbA2% MEASURED VALUE

# FIG.7

# FIG.8

TRANSITION OF DEGREE OF SEPARATION

# FIG.9

FIG.10

Europäisches
Patentamt

European
Patent Office

Office européen
des brevets

**EUROPEAN SEARCH REPORT**

Application Number

EP 24 17 1152

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/034563 A1 (YOTANI TAKUYA [JP]) 6 February 2014 (2014-02-06) * paragraph [0002] - paragraph [0003] * * paragraphs [0033], [0035], [0037] * | 1,3,5,9 | INV. G01N33/48 G01N33/72 G01N30/50 |
| X | JP 2011 242238 A (TOSOH CORP) 1 December 2011 (2011-12-01) * paragraphs [0003], [0028] - paragraph [0029]; figure 2 * * paragraph [0032] - paragraph [0035]; figure 4 * | 1,3,9,10 | ADD. G01N30/88 |
| X | JP 2001 221789 A (SEKISUI CHEMICAL CO LTD) 17 August 2001 (2001-08-17) * paragraph [0008] - paragraph [0009] * * paragraph [0060] * | 1,3-5, 7-9 | |
| X | EP 1 103 812 B1 (SEKISUI CHEMICAL CO LTD [JP]) 18 November 2015 (2015-11-18) * paragraph [0135] - paragraph [0138] * * paragraph [0040] * * paragraph [0099]; figure 16 * | 1,2,6,9 | |

TECHNICAL FIELDS
SEARCHED (IPC)

G01N

The present search report has been drawn up for all claims

| Place of search | Date of completion of the search | Examiner |
|---|---|---|
| Munich | 23 September 2024 | Gomez, Adriana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 17 1152

This annex lists the patent family members relating to the patent documents cited in the above-mentioned European search report.
The members are as contained in the European Patent Office EDP file on
The European Patent Office is in no way liable for these particulars which are merely given for the purpose of information.

23-09-2024

| Patent document cited in search report | | Publication date | Patent family member(s) | | Publication date |
|---|---|---|---|---|---|
| US 2014034563 | A1 | 06-02-2014 | CN | 101978262 A | 16-02-2011 |
| | | | EP | 2261655 A1 | 15-12-2010 |
| | | | JP | 5167344 B2 | 21-03-2013 |
| | | | JP | 6227689 B2 | 08-11-2017 |
| | | | JP | 6876669 B2 | 26-05-2021 |
| | | | JP | 2013019918 A | 31-01-2013 |
| | | | JP | 2014157165 A | 28-08-2014 |
| | | | JP | 2016128836 A | 14-07-2016 |
| | | | JP | 2018028547 A | 22-02-2018 |
| | | | JP | 2019032346 A | 28-02-2019 |
| | | | JP | WO2009123199 A1 | 28-07-2011 |
| | | | US | 2011073539 A1 | 31-03-2011 |
| | | | US | 2012067823 A1 | 22-03-2012 |
| | | | US | 2014034563 A1 | 06-02-2014 |
| | | | WO | 2009123199 A1 | 08-10-2009 |
| JP 2011242238 | A | 01-12-2011 | JP | 5521218 B2 | 11-06-2014 |
| | | | JP | 2011242238 A | 01-12-2011 |
| JP 2001221789 | A | 17-08-2001 | JP | 4404429 B2 | 27-01-2010 |
| | | | JP | 2001221789 A | 17-08-2001 |
| EP 1103812 | B1 | 18-11-2015 | AU | 5064499 A | 28-02-2000 |
| | | | CA | 2339360 A1 | 17-02-2000 |
| | | | EP | 1103812 A1 | 30-05-2001 |
| | | | ES | 2563162 T3 | 11-03-2016 |
| | | | US | 6428704 B1 | 06-08-2002 |
| | | | WO | 0008460 A1 | 17-02-2000 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017227461 A